(19) Europäisches Patentamt
European Patent Office
Office européen des brevets

(11) EP 0 864 361 A2

(12) # EUROPÄISCHE PATENTANMELDUNG

(43) Veröffentlichungstag:
**16.09.1998 Patentblatt 1998/38**

(21) Anmeldenummer: **98104128.8**

(22) Anmeldetag: **09.03.1998**

(51) Int. Cl.[6]: **B01J 31/04**, C07C 51/41, C08G 64/34
// (B01J31/04, 103:20)

(84) Benannte Vertragsstaaten:
**AT BE CH DE DK ES FI FR GB GR IE IT LI LU MC NL PT**
Benannte Erstreckungsstaaten:
**AL LT LV MK RO SI**

(30) Priorität: **12.03.1997 DE 19710158**

(71) Anmelder:
**Buna Sow Leuna Olefinverbund GmbH
06258 Schkopau (DE)**

(72) Erfinder:
- **Eckert, Rolf, Dr.
06130 Halle (DE)**
- **Feix, Gunter, Dr.
06122 Halle (DE)**
- **Schimmel, Karl-Heinz, Dr.
06116 Halle (DE)**
- **Adler, Bernhard, Prof. Dr.
06132 Halle (DE)**

(54) **Polyzinkcarboxylat-Katalysatoren zur Herstellung von Polyalkylencarbonaten**

(57) Die Erfindung betrifft Polyzinkcarboxylat-Katalysatoren, die durch Umsetzung von Zinkdiethyl mit Dicarbonsäuren unter anaeroben Bedingungen hergestellt werden. Der Einsatz dieser Katalysatoren liefert bei anaerober Polymerisation und ohne Verwendung weiterer Lösungsmittel Polyalkylencarbonate, die frei von Polyolen als Nebenprodukte herkömmlicher Katalysatoren sind.

EP 0 864 361 A2

## Beschreibung

Die Erfindung betrifft Polyzinkcarboxylate, die als Katalysatoren zur Synthese von Polyalkylencarbonaten eingesetzt werden können.

Bekannt ist, daß Katalysatoren zur Synthese von Polyalkylencarbonaten entweder durch Umsetzung von Zinkoxiden bzw. Zinkhydroxiden mit Dicarbonsäuren zu polymeren Polyzinkcarboxylaten gemäß Gl. 1 hergestellt werden (US 4.943.677, US 5.026.676, EP 0.358.326, US 4.960.862):

$$\left\{\begin{matrix} ZnO \\ Zn(OH)_2 \end{matrix}\right\}_n + n\ HOOCRCOOH \longrightarrow -(OZnOC{=}ORC{=}O\text{-})_n + n\ H_2O \qquad \text{(Gl.1)}$$

$$R = (CH_2)_k \quad \text{mit:} \quad 0 <= k <= 8$$

oder durch Umsetzung von Zinkdiethyl mit Wasser gemäß Gl. 2 gebildet werden:

$$4\ Et_2Zn + 4H_2O \rightarrow EtZn(OZn)_3OH + 7EtH \qquad \text{(Gl. 2)}$$

Et = Ethylgruppe (M. RÄTSCH, W. HAUBOLD: Faserforschung u. Textiltechnik 28 (1977)15-21).

Bekannt ist ferner, daß sowohl für die Herstellung des Katalysators als auch für die Polymerisation aprotische Lösungsmittel empfohlen werden (US-Patent 5 026 676 bzw. J.M.BRONK, J.S.RIFFLE: Polym. Prepr. Am. Chem. Soc. Div. Polym. Chem. 35 (1994) 815-16). Das Fernhalten protischer Lösungsmittel im Polymerisationsprozeß einerseits steht mit der Katalysatorherstellung unter Verwendung oder Freisetzung des protischen Lösungsmittels Wasser als Reaktant oder Reaktionsprodukt gemäß Gl.1 bzw. 2 andererseits im Widerspruch und ist die Ursache u.a. für eine unerwünschte Nebenreaktion während des Polymerprozesses, der Polyolbildung. Zur Vermeidung dieser Nebenreaktion muß prinzipiell eine Katalysatorherstellung, die frei von der Verwendung oder Bildung von $H_2O$ ist, angewendet werden.

Den wissenschaftlichen Beweis einer Selektivitätsminderung des Katalysators durch das bei seiner Bildung mit anwesende Wasser kann wie folgt geführt werden. Beobachtet man gravimetrisch das bei der Katalysatorbildung freigesetzte Wasser in Abhängigkeit von der Zeit, so zeigt sich, daß sowohl beim Zinkglutarat als auch -adipat nur ein Bruchteil des Erwartungswertes auch tatsächlich freigesetzt wird (Abb.1).

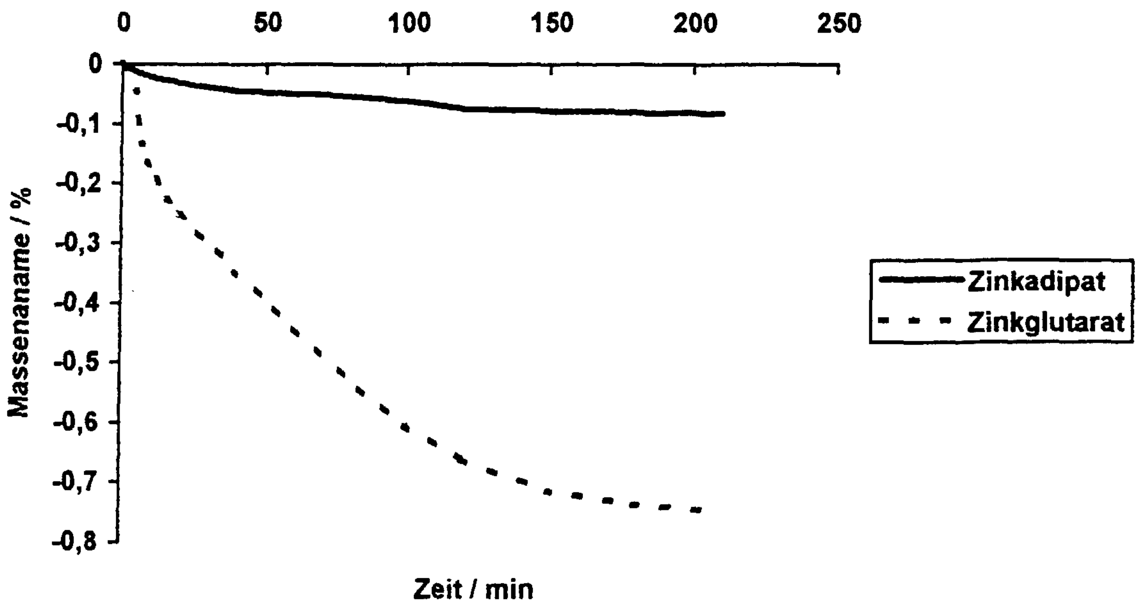


Abbildung1: Freisetzen des Reaktionswassers gemäß Gl. 1 in Abhängigkeit der Z

Der überwiegende Teil verbleibt koordinativ am Zink gebunden, also am Katalysator. Das Auftreten der Zn...$OH_2$-rocking-Schwingung bei 834 $cm^{-1}$ bzw. der Zn...$OH_2$-wagging-Schwingung bei 505 $cm^{-1}$ sind u.a. ein Indiz für diese Koordination (Tab. 1, 1. und 3. Zeile). Laugt man darüber hinaus einen gemäß Gl.1 hergestellten Kontakt mit Wasser, so zeigt die in Wasser lösliche Modifikation des Kontaktes ein starkes Anwachsen der Zn...$OH_2$-rocking-Schwingungen (Tabelle 1, 1. Zeile, 6.Spalte). Diese Modifikation des Katalysators ist zudem völlig unwirksam.

Tabelle1

| IR-Signalintensitäten von Katalysatorbanden in Abhängigkeit von der Präparationsart | | | | | | |
|---|---|---|---|---|---|---|
| **Signalart** | **Wellenzahl in $cm^{-1}$** | **Fällung[1)]** | **Präparation nach Gl. 3** | **Rückstand nach Laugung** | **Filtrat der Laugung** | **Kontakt nach tribochem. Präparation** |
| rZn...$OH_2$/r$CH_2$ | 834/767 | 1 | 0,17 | 0,76 | 1.11 | 0,68 |
| r$CH_2$/d$CH_2$ | 767/1458 | 0,33 | > 0,25 | 0.30 | 0,36 | 0,31 |
| d$CH_2$/wZn...$OH_2$ | 767/505 | 0,07 | > 0 | 0,055 | 0,12 | 0.055 |

[1)] von $ZnCl_2$ mit Natriumglutarat im Wasser

Dabei kann das Anwachsen der $rZnOH_2$-Schwingung als direktes Maß für die Selektivitätsminderung des Kontaktes genutzt werden. Daß heißt, die Synthese des Katalysators sollte prinzipiell unter Abwesenheit von Wasser erfolgen.

Es besteht die Aufgabe, Polyzinkcarboxylat-Katalysatoren zu entwickeln, die hoch selektiv Polyalkylencarbonate ohne Nebenprodukte von Polyolen bilden können. Die Polymerisationssynthese soll dabei ohne Verwendung eines zusätzlichen Lösungsmittels erfolgen.

Erfindungsgemäß wird die Aufgabenstellung einer $H_2O$-freien Polyzinkcarboxylatsynthese durch den Umsatz von Zinkdiethyl mit Dicarbonsäuren unter anaeroben Bedingungen gelöst:

$$n\ Et_2Zn + n\ HOOCRCOOH \rightarrow -(ZnOC{=}ORC{=}OO)-_n + 2nEtH \quad Et = C_2H_5 \quad R = (CH2)_k \text{ mit: } 0<=k<=8 \qquad \text{Gl.3}$$

Im Unterschied zur Reaktion gemäß Gl.1 entsteht bei dieser Synthese als Nebenprodukt nicht Wasser, sondern

Ethan. Dieses Nebenprodukt führt im status nascendi darüber hinaus zu einer im Vergleich zu herkömmlich hergestellten Katalysatoren 4fach so großen Oberfläche. So beträgt die Oberfläche eines nach Mahlen in der Schwingmühle hergestellten Kontaktes ca. $6m^2/g$, das aus Zinkdiethyl hergestellte Produkt besitzt eine Oberfläche von ca. $27m^2/g$. Bei der Synthese nach Gl. 3 ist jedoch zu beachten, daß am Syntheseende keine freie Dicarbonsäure mehr vorhanden ist, denn auch sie würde Anlaß zur Polyglycolbildung geben. Man arbeitet deshalb bei der Katalysatorsynthese nicht mit äquimolaren Reaktantmengen, sondern mit einem geringfügigen Überschuß an Zinkdiethyl. Dieses wird teilweise vom Polyzinkglutarat adsorptiv festgehalten. Ultraspuren von $H_2O$ in den Reaktantgasen führen dann nicht sofort zu einer Desaktivierung des Kontaktes. Es bildet sich vielmehr gemäß Gl. 2 eine zusätzlich katalytisch wirkende Substanz.

Als Dioarbonsäuren werden vorzugsweise Glutar- oder Fumarsäure eingesetzt.

Im Unterschied zu herkömmlichen Verfahrensweisen wird anschließend die Polymerisation nicht mit einem Lösungsmittel, sondern allein mit überkritischem $CO_2$ durchgeführt. Während protisch polare Lösungsmittel unmittelbar die Selektivität des Kontaktes vermindern oder ihn völlig desaktivieren können, wirken aprotisch unpolare Lösungsmittel indirekt desaktivierend. Denn durch die Reaktantgase eingeschleppte, nicht vermeidbare Ultraspuren von Wasser werden durch zwischenmolekulare Wechselwirkungen aus dem Lösungsmittel bevorzugt an die Oberfläche und damit auch an die Katalysatoroberfläche gedrängt und verändern ihn, wie oben beschrieben. Bei der unter anaeroben Bedingungen durchgeführten Reaktion entstehen dagegen dann polyglycolfreie Polyalkylencarbonate, wenn die Umsetzung gemäß Gl. 3 vollständig verlief, d.h., der Katalysator keine Verunreinigungen an freier Dicarbonsäure enthält.

## Beispiel

### Herstellung des Katalysators

Glutarsäure wird in wasserfreiem Diethylether gelöst und unter Rühren tropfenweise gekühltes Zinkdiethyl unter anaeroben Bedingungen zugegeben. Zum Ausreagieren kann anschließend leicht erwärmt werden. Die Reaktion gilt als beendet, wenn keine freie Glutarsäure mehr analytisch nachweisbar ist. Nach Absaugen des Diethylethers im Vakuum kann der Kontakt ohne weitere Aufarbeitungsschritte zur Polymerisation verwendet werden. Seine Selektivität ist am Bandenverhältnis der IR-Schwingungen bei 834/768 $cm^{-1}$zu prüfen. Es muß kleiner als 0,2 sein. Im vorliegenden Falle beträgt es z. B. 0,17 (Tab.1, Spalte 4).

### Austestung des Katalysators

Die Austestung des Katalysators erfolgt mittels SFE (Superkritische-Fluid-Extraktion) bei einer Reaktionstemperatur von 80 °C, bei einem Druck von ca. 100 bar und einer Reaktionszeit von 4h ohne ein weiteres Lösungsmittel. Der Kontakt wurde in vorgelegtem Propylenoxid suspendiert.

Tabelle 2

| Simulationsversuche zur Austestung von Katalysatoren | | | |
|---|---|---|---|
| **Katalysatorherstellung** | **% PPC[1)]** | **%PG[2)]** | **%PC[3)4)]** |
| tribochemisch | 66 | 29 | 5 |
| azeotrope Toluendestillation | 79 | 17 | 4 |
| nach Gl.3 | 96 | 0 | 4 |

[1)] PPC Polypropylencarbonat,
[2)] PG Polyglycol ,
[3)] PC Propylencarbonat (monomer),
[4)]Mittelwerte

### Oberflächenbestimmung

Die Oberfläche des Kontaktmaterials wurde durch $N_2$-Adsorption mit dem Areometer von STRÖHLEIN-Instruments bestimmt.

## Patentansprüche

1. Polyzinkcarboxylat-Katalysatoren zur Herstellung von Polyalkylencarbonaten dadurch gekennzeichnet, daß diese aus halatopolymeren Zinkcarboxylaten bestehen, die durch Umsatz von Zinkdiethyl mit aliphatischen Dicarbonsäuren unter anaeroben Bedingungen und einem solchen Überschuß an Zinkdiethyl gebildet werden, daß keine freie Dicarbonsäure mehr vorliegt, daß dieser Katalysator bei der Polymerisation ohne Verwendung eines zusätzlichen Lösungsmittels, nur in überkritischem Kohlendioxid bzw. Ethylen- oder Propylenoxid mit hoher Selektivität polyolfreie Polyalkylencarbonate liefert und daß das bei der Katalysatorreaktion freigesetzte Ethan ein Halatopolymer hoher Oberfläche liefert.

2. Polyzinkcarboxylat-Katalysatoren nach Anspruch 1, dadurch gekennzeichnet, daß als Dicarbonsäuren Glutar- oder Fumarsäure eingesetzt wird.